(19) Europäisches Patentamt
European Patent Office
Office européen des brevets

(11) Publication number: 0 284 536 A1

# (12) EUROPEAN PATENT APPLICATION

(21) Application number: 88500016.6

(22) Date of filing: 18.02.88

(51) Int. Cl.4: C 07 D 277/48

(30) Priority: 20.02.87 ES 8700719

(43) Date of publication of application: 28.09.88 Bulletin 88/39

(84) Designated Contracting States: DE ES IT

(71) Applicant: CENTRO MARGA PARA LA INVESTIGACION S.A.
Muntaner 212, 5o-510
E-08036 Barcelona (ES)

(72) Inventor: Ballester Rodes, Montserrat
Marqués de Santa Ana No 14, 2a
E-08023 Barcelona (ES)

Palomo Nicolau, Francisco Eugenio
Dos de mayo No 34, 2o-1a
E-08190 Sant Cugat del Valles Barcelona (ES)

Palomo Coll, Antonio Luis
Escuelas Pias No 18, 5o-1a
E-08017 Barcelona (ES)

(74) Representative: Ballester Rodés, Albert
Muntaner 212, 5o- 508
E-08036 Barcelona (ES)

## (54) A process for the preparation of 3-(2-guanidinothiazol-4-yl-methylthio)propionitrile.

(57) The present invention describes a beta-cyanoethylation process of thiol-compounds by acrylonitrile, for the preparation of 3-(2-guanidinothiazol-4-yl-methylthio)propionitrile, in an aqueous-methanolic medium.

EP 0 284 536 A1

**Description**

A process for the preparation of 3-(2-guanidinothiazol-4-yl-methylthio)propionitrile

It is known (V.Migrdichian; Chemistry of Organic Cyanogen Compounds, Pub.Corp. New York, 1947) that the nitrile group experiments the transformation into thioamides, iminoethers or imidates, and amidines; in the acrylonitrile case, 3-alcohoxy-propionitrile is also formed in the presence of alcohols and inorganic or organic alkaline bases. The formation of imidates is also described in detail (S.R.-Sandler, W.Karo; Organic Functional Group Preparations 12-III, Academic Press, New York 1972, pag. 268) and it happens, by treatment with hydrogen chloride. This yields easily the 3-chloropropionitrile and its 3-chloropropionimidates.

The use of 3-chloropropionitrile is described in the patents GB 2052478A and the spanish ones 544597 and 549312; and the use of the 3-methyl-mercaptopropionitrile in the U.S. patent 4.496.737.

In the case of the patent GB 2052478A, the 3-chloropropionitrile is used to prepare the 3-(2-aminothiazol-4-yl-methylthio)propionitrile.

In the case of the previously mentioned spanish patents, it is generated "in situ" in the reaction medium, the conversion of the 1-amino-3-chloro-propyliden-sulphamide in N-sulfamylacrylamidine, described in the U.S. patent-4.496.737 being prepared from a laborious and expensive process. In both cases the aim consists of the reaction of those syntons with the S-(2-guanidino-4-thiazolylmethyl)- isothiourea or the thiol derivative.

A new process has been discovered now, by means of which the acrilonitrile actuates as scavenger of the thiolate group, that is generated from a 5-(2-substituted-4-thiazolylmethyl)-thiol, S-(2-substituted-4-thiazolylmethyl)-isothiourea, S-(2-substituted-4-thiazolylmethyl)-phosphorothiolate, S-(2-guanidino-4-thiazolylmethyl)-amidinoisothiourea or the dithiocarbamate.

The present invention consists in a new process for the preparation of 3-(2-guanidinothiazol-4-yl-methylthio) propionitrile, where a S-(2-substituted-4-thiazolylmethyl)-thio-derivative is reacted with acrylonitrile, in the presence of hydroxile ions. In the case that the substitute $R^1$ may be the guanidino group, the desired product is obtained.

The substitute $R^1$ can be represented by other groups as amino, cyanamido or chlorine, which respectivelly are reacted with cyanamide, ammonium chloride or guanidine.

The process of the invention is represented by the following scheme of reaction, where the preferred reagents are clearly shown, illustrating the process of this invention:

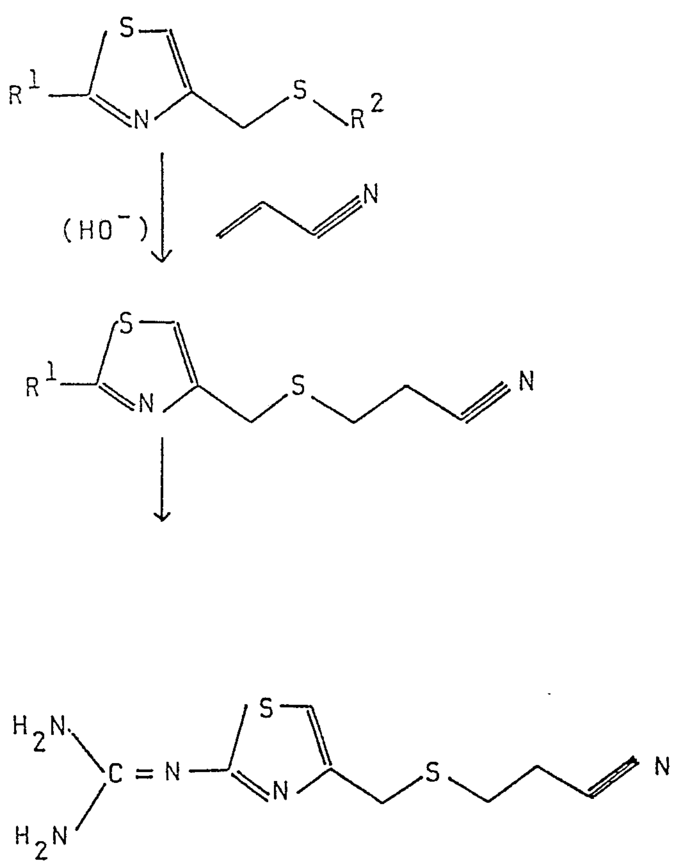

$R^2$ can represent an hydrogen atom or a group that in a basic medium liberates the thiolate or mercaptane function. The structure of such group can be selected preferably among the following:

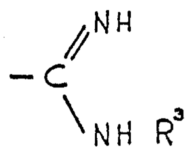 , 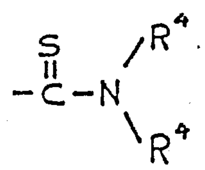

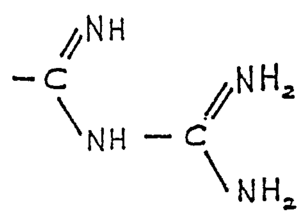 , 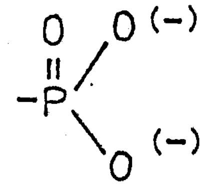

where $R^3$ represents an hydrogen atom and $R^3$ as $R^4$ an alkyl group of low molecular weight having from one to four carbon atoms, preferably two, or a ring as the one of the morpholine or piperidine, incorporating the nitrogen atom of the previously expressed radicals.

As it is known, the thiol group is very easily oxidable to disulphide, particularly in the presence of ambiental oxygen and in an alkaline medium. That is why, and according to the previously expressed scheme, one of the preferred methods consists in the reaction of the acrylonitrile with the thio-compound, $R^2$ being one of the groups previously mentioned, from where the thiol-derivative is generated, being catched by the acrylonitrile to form the desired product.

The generation or formation of the thiol function, from the thio-derivatives, is suitably described by Wardell (The Chemistry of thiol groups, Part.1; Ed. Saul Patai, John Willey New York 1974, pag. 163). The derivatives of S-(2-substituted-4-thiazolylmethyl)-isothiourea were prepared applying the methods described by Ziegler and collab. (J.Am.Chem.Soc.,68, 2155, 1946), Yellin and Collab. (Spanish Pat.468985 of I.C.I.). The reaction between 1,3-dichloroacetone successively with N-cyanothiourea and thiourea, or with two equivalents of amidinothiourea, yields respectivelly S-(2-cyanamido-4-thiazolylmethyl)-isothiourea or S-(2-guanidino-4-thiazolylmethyl)-amidinoisothiourea dihydrochloride. From the reaction between 1-chloro-3-hydroxyacetone and thioformamide, it resulted the 2-hydroxy-4-chloromethylthiazol that with the reagent dimethylformiminium chlorosulphide chloride produces 2-chloro-4-chloromethylthiazol. From the reaction between 1-chloro-3-hydroxyacetone and amidinothiourea, it was prepared 2-guanidino-4-hydroxymethyl-thiazol, and with the thiourea the corresponding 2-amino-4-hydroxymethyl-thiazol. All those compounds are used for the invention purposes, without requiring previous treatments of purification , and for instance in the case of the isothiouronium compounds, no isolation is required, this fact being adventageous as a technologic alternative of the process.

The preparation of phosphorothiolates was realized according to the methods of Piper and Johnston (J.Org.Chem.,32,-1261,1967) or the one of Akerfeldt (Acta Chem. Scand. 14, 1980, 1960). With the dithiocarbamates and its basic hydrolysis, it was used the Kulka process (Cam.J.Chem.34, 1093, 1956).

The equimolecular reaction of 1,3-dichloroacetone, first with N-diethyl-thiocarbamate and afterwards with N-formamidinothiourea (amidinothiourea) yields the S-(2-guanidino-4- thiazolylmethyl)-N-diethylthiocarbamate hydrochloride. In a similar way, first with phosphorothiolate and after with N-formamidinothiourea, S-(2-guanidino-4-thiazolilmethyl)-phosphorothiolate is obtained.

It is surprising the efficacity that the acetonitrile has demonstrated, to react with 2-substituted-thiazol-4-ylmethylthiols, generated in situ, in the presence of hydroxile ions. These ions proceed from the incorporation in the reaction medium, of basic compounds as, hydroxides of alkaline metals and of calcium, carbonates and alkaline bicarbonates, disodic and trisodic phosphates. Among the organic compounds, tertiary amines as the trietylamine, tripropylamine, n-ethylpiperidine, n-ethylmorpholine, bicyclic amidines as DBU and DBN (abbreviations adopted in the usual terminology).

The reaction medium is constituted by aqueous-alcoholic solutions of methanol, ethanol and isopropanol. Methanol is preferably desired, where the prepared isothiouronium compound does not need isolation; however, the isopropanol is desirable, that though it causes a precipitate, gets redissolved by the addition of enough quantity of methanol or water.

Example 1

The solution of 60.6 g (0.2 mol) of S-(2-guanidine-4-thiazolylmethyl)-isothiourea dihydrochloride and 28.5 ml(0.45 mol) of acrylonitrile in 100 ml of methanol, with 60 ml of water, is vigorously stirred, refrigerating with a water bath. 13.4 g (0.355 mol) of sodium hydroxide (98-99%) dissolved in 40 ml of water are added. The temperature is controlled between 20 and 25°C. In about 30 minutes it is reached the PH = 14.20. By gradual addition of acetic acid (6.0 ml) it is carried to the end of the reaction at PH = 8.05. The end is controlled by nitroprussiate assay: to 2 ml of mixture it is added a nitroprussiate solution (0.5 ml, 1% in water), little crystals of sodium chloride and three drops of concentrated ammonia; the apparition of an intense blue-violet colour indicates the presence of isothiouronium compound and it is continued until an absolute negative test. The virtually quantitative transformation happens between 45-90 minutes. Afterwards, it is added 0.1 mol of hydrogen chloride and the methanol is evaporated at reduced pressure; the liquids are extracted with dichloromethane and the aqueous phase is brought to PH = 9.0, cooled and filtered yielding 43.44 g of the title's compound with m.p.: 131-132°C and 90% yield with respect to the theoretical.

The aqueous liquids of filtration in an acidic medium (acetic) in the presence of sodium nitrite, produce emission of nitrogen, proceding from the urea.

Example 2

To the solution of 35.65 g (0.15 mol) of 2-guanidino-4-thiolmethylthiazol hydrochloride in a mixture of 100 ml of methanol with 100 ml of water, it is added first 14.25 ml (0.225 mol) of acrylonitrile and afterwards at the temperature of 5°C, sodium hydroxide 98-99% (9.0 g, about 0.22 mol). It is continued as in the first example isolating the title's compound with a similar yield.

Preparation of 2-guanidino-4-thiol-methylthiazol hydrochloride:

To the recently prepared 20% solution of sodium methoxide (89.8 g; 0.33 mol), and cooled at 0°C, a hydrogen sulphide flow is got through, until saturation (approximately 30-60 minutes), and without stopping the gas flow, it is gradually added (4 hours) and with energetic stirring, the 2-guanidino-4-chloromethyl-thiazol hydrochloride (34.10 g, 0.15 mol) in 125 ml of methanol. Afterwards it is continued during 60 minutes more and after it is brought to acid pH with a solution of hydrogen chloride in isopropanol. The sodium hydrochloride is filtered and brought to dryness, yielding the mercapto compound that is directly used.

Preparation of 2-guanidino-4-chloromethyl-thiazol hydrochloride:

To obtain 0.2 mol of dimethylformiminium chlorosulphide chloride, 28.4 g of thionile chloride are added to the solution of 18.74 g of dimethylformamide in 40 ml of benzene, controlling that the temperature does not exceed 28-30°C. The lower phase is added gradually to the suspension of 31.30 (0.15 mol) of 2-guanidino-4-hydroxymethyl-thiazol hydrochloride in 115 ml of dichloromethane at room temperature. Afterwards, it is maintained with stirring at 35°C for 2 hours, cooled at 20°C, filtered, washed with dichloromethane and dried, yielding 31.34 g of 2-guanidino-4-chloromethyl-thiazol hydrochloride, with mp.: 191-193°C and 92.0% yield with respect to the theoretical.

Example 3

To a solution of 19.93 g (0.1 mol) of 3-(2-amino-4-yl-methylthio) propionitrile in 80 ml of water containing 3.6 g of hydrogen chloride, it is added 8.4 g (0.2 mol) of cyanamide and it is heated at 85°C during 120 minutes. Next, it is brought to alkaline PH and cooled down to 5°C. 18.2 g of the title's compound are isolated, with a mp.: 132°C (Acetone) with 75.4% yield with respect to the theoretical.

Preparation of 3-(2-amino-4-yl-methylthio)propionitrile:

Following the first example and substituting the 2-guanidino by the equivalent amount of S-(2-amino-4-thiazolylmethyl)-isothiourea, the reaction is controlled in the same conditions. The aqueous solution resulting from the evaporation of the methanol and at a alkaline PH, is extracted with chloroform; the organic phase, dried and concentrated at reduced pressure yields 35.8 g of 3-(2-amino-4-yl-methylthio)propionitrile with mp.: 104-106°C and similar yield (89.8%).

Example 4

Following the second example and substituting the 2-guanidino-4-thiolmethylthiazol by 24.85 g (0.15 mol) of 2-chloro-4-thiolmethyl-thiazol, it is made to react first with 14.25 ml of acrylonitrile and after at pH = 8.0 with 12.0 g (0.20 mol) of guanidine dissolved in 50 ml of methanol heating at reflux temperature during 60 minutes.

For the isolation, it is proceeded as in the second example, resulting the title's compound, 28.0 g with the same melting point and with a 77.4% yield with respect to the theoretical.

Example 5

The solution of 2.24 g (0.01 mol) of 3-(2-cyanamidothiazol-4-yl-methylthio)propionitrile in 50 ml of methanol with 1.0 g of ammonium chloride, is heated at reflux temperature during 60 minutes. The end of the reaction is controlled by thin layer chromatography. The solvent is evaporated at reduced pressure, it is added water (10 ml) and cooled down to 0-5°C; adjusted at alkaline PH, 2.13 g of title's compound are isolated with m.p.: 130-132°C and yield 88.2% with respect to theoretical.

The previous cyanamido-thiazol derivative, is prepared by reaction of S-(2-cyanamido-4-thiazolylmethyl)isothiourea with acrylonitrile following the first example.

Example 6

Following the first example and substituting the sodium hydroxide by 1,8-diazabicyclo[5.4.0]undec-7-ene (DBU), the title's compound is obtained with a similar yield.

Example 7

Following the second example and substituting the sodium hydroxide indistinctly by triethylamine or N-ethylpiperidine, the title's compound is obtained with a similar yield.

Example 8

Following the first example and substituting the S-(2-guanidino-4-thiazolylmethyl)isothiourea by 69.05 g (0.2 mol) of S-(2-guanidino-4-thiazolylmethyl)amidinoisothiourea hydrochloride, the title's compound is obtained with similar yield.

Example 9

Following the first example and substituting the methanol-water by isopropanol-water, the title's compound is obtained with a similar yield.

Example 10

To 100 ml of methanol, are added first 45.4 g (0.2 mol) of 2-guanidino-4-chloromethylthiazol hydrochloride and later 15.22 g (0.2 mol) of thiourea. It is heated at 60° C and stirrer during about 60 minutes. Next, it is cooled down at room temperature and 60 ml of water are added, continuing as specified in the first example. The title's compound is obtained with the same characteristics, similar yield and mp. : 132° C.

Example 11

During 60 minutes, it's heated at reflux temperature a solution of 22.7g (0.1 mol) of 2-guanidino-4-chloromethyl thiazol hydrochloride and 18.00 g (0.1 mol) of sodium phosphorothiolate, in 60 ml of methanol and 40 ml of water. Afterwards, it is cooled at 35-40° C, and 14.20 ml (0.22 mol) of acrylonitrile and 10.0 g of sodium hydroxide are added. The mixture is maintained with agitation at this temperature until negative nitroprussiate test. For the isolation, it is proceeded according to the first example, isolating 20.60g of the title's compound with a 77.0% yield with respect to the theoretical.

Example 12

Following the example 10 and substituting the thiourea by the equivalent amount of sodium N-diethylthiocarbamate (34.50 g, 0.2 mol) and the methanol by the isopropanol, the mixture, practically neutralized, is heated at reflux temperature. Then, it is added an equivalent of sodium hydroxide being maintained with agitation at 45° C. Finally, it is proceeded as described in the first example, to isolate the title's compound.

**Claims**

1. A process for the preparation of 3-(2-guanidinothiazol-4-yl-methylthio)propionitrile, characterized because the acrylonitrile and a compound of the following formula:

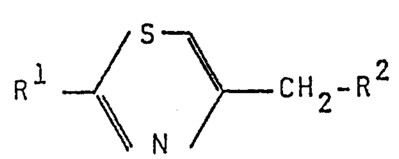

where : $R^1$ is a group selected from the amino, cyanamido chlorine, guanidino, and $R^2$ may be a thiol, isothiurea, amidinoisothiurea, phosphorothiolate or N-diethylthiocarbamate are made to react in an aqueous-methanolic, aqueous-ethanolic or aqueous-isopropanolic medium, at temperatures comprised from room temperature to 60° C, in the presence of a basic compound, inorganic or organic, aliphatic or heterobicyclic, of low molecular weight and, being $R^1$ an amino group is made to react with cyanamide, if $R^1$ is cyanamide is made to react with ammonium chloride and if $R^1$ is a chlorine atom, is made to react with guanidine, to obtain a compound of the following formula:

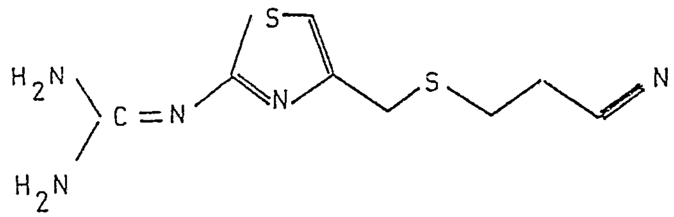

2. A process according to the first claim, characterized because a basic compound is selected from the sodium hydroxide, potassium hydroxide, sodium bicarbonate, sodium carbonate, potassium bicarbonate, trisodium phosphate, triethylamine, N-ethylpiperidine, 1,8-diazabicyclico[5.4.0]undec-7-ene and 1,5-diazabicyclo[4.3.0]non-5-ene.

3. A process for the preparation of 3(2-guanidinothiazol-4-yl-methylthio)propionitrile.

European Patent Office

# EUROPEAN SEARCH REPORT

Application Number

EP 88 50 0016

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | JOURNAL OF MEDICINAL CHEMISTRY, vol. 27, no. 27, July 1984, pages 849-857, American Chemical Society, Washington, DC, US; I. YANAGISAWA et al.: "Histamine H2 receptor antagonists. 1. Synthesis of N-Cyano and N-Carbamoyl Amidine Derivatives and their biological activities. * Pages 849-851 * --- | 1 | C 07 D 277/48 |
| A | FR-A-2 450 827 (YAMANOUCHI) ----- | 1 | |
| | | | TECHNICAL FIELDS SEARCHED (Int. Cl.4) |
| | | | C 07 D 277/00 |

The present search report has been drawn up for all claims

| Place of search | Date of completion of the search | Examiner |
|---|---|---|
| THE HAGUE | 27-05-1988 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document